# EUROPÄISCHE PATENTSCHRIFT

(11) **EP 2 568 930 B1**
(45) Veröffentlichungstag und Bekanntmachung des Hinweises auf die Patenterteilung: **03.06.2015**
(21) Anmeldenummer: 11718324.4
(22) Anmeldetag: 20.04.2011
(51) Int. Cl.: A61F 5/01, B29C 70/54

(54) **ORTHESE UND HERSTELLUNGSVERFAHREN**
ORTHOSIS AND PRODUCTION METHOD
ORTHÈSE ET PROCÉDÉ DE FABRICATION

(30) Priorität: 11.05.2010 DE 102010020259
(43) Veröffentlichungstag der Anmeldung: 20.03.2013
(73) Patentinhaber: Wilhelm Julius Teufel GmbH, 73117 Wangen (DE)
(72) Erfinder: THIEL, Christoph, 73630 Remshalden-Rohrbronn (DE); BLUM, Thomas, 65199 Wiesbaden (DE)
(74) Vertreter: DREISS Patentanwälte PartG mbB
(86) Internationale Anmeldenummer: PCT/EP2011/056313
(87) Internationale Veröffentlichungsnummer: WO 2011/141283

(56) Entgegenhaltungen:
- WO-A1-97/16140
- US-A- 5 826 304
- US-A- 6 146 344
- US-A1- 2001 031 935
- US-B1- 6 171 535

## Beschreibung

### Orthese und Herstellungsverfahren

Die Erfindung betrifft eine Orthese sowie ein Verfahren zu deren Herstellung mit einem ersten und zumindest einem weiteren Stützabschnitt, die zumindest einen räumlich geformten Stützkörper bilden, wobei die Stützabschnitte zur Anlage am Patienten ausgebildet und zur Stützung von Gliedmaßen oder Körperabschnitten am Patienten ausgebildet sind.

Eine solche Orthese kann beispielsweise als Knöchel-Fuß-Orthese ausgebildet sein. Eine solche Orthese wird bei Personen mit starkem Hängefuß verwendet oder von Personen, welche unter einer Muskelschwäche in den unteren Extremitäten leiden. Diese Orthesen umfassen einen ersten Stützabschnitt, der beispielsweise eine Fußsohle oder eine Fußplatte bildet, sowie einen weiteren Stützabschnitt, welcher am Unterschenkel angreift. Durch die Verbindung der beiden Stützabschnitte über einen Verbindungsabschnitt wird ein fester Stützkörper gebildet, der die Person beim Gehen unterstützt.

Eine solche Fußorthese ist beispielsweise aus der DE 697 32 541 T2 bekannt. Zwischen einer Fußsohle als erster Stützabschnitt und einer Schienbeinanlage als zweiter Stützabschnitt ist ein Verbindungselement vorgesehen, welches die beiden Stützabschnitte miteinander verbindet. Insbesondere im Übergangsbereich zwischen der Fußsohle und dem am Schienbein anliegenden Stützabschnitt ist ein Bügel ausgebildet, welcher aus mehreren kohlefaserverstärkten Lagen eines Kohlefasergewebes besteht, das mit einer Epoxidgrundmasse getränkt ist. Vom Bügel aus erstrecken sich mehrere Lagen von Kohlefasergeweben in unterschiedli-cher Länge in die Fußsohle hinein, wobei die Kohlefasergewebe in Strei-fen ausgebildet sind und in unterschiedlichen Richtungen zumindest teilweise umgelenkt werden, um sich zum Fußballen und zur Fußsohle hin zu erstrecken. Diese Anordnung gewährt zwar eine Haltbarkeit und Steifigkeit im Übergangsbereich zwischen dem Bügel und der Fußsohle. Dies weist jedoch den Nachteil auf, dass der Tragekomfort aufgrund der hohen Steifigkeit vermindert ist. Darüber hinaus erfolgt eine erhebliche Verdickung des Bügels. Dies wirkt sich nachteilig für den Patienten aus, da im Bereich des medialen und lateralen Verlaufs c er Spange zur Fußsohle im Schuh sehr wenig Raum verbleibt und durch die Aufdickung eine starke Aufweitung des Schuhs erfolgt.

Eine ähnliche Ausgestaltung geht beispielsweise aus der DE 603 15 698 T2 hervor. Durch die verschiedenlagige Anordnung einzelner Gewebelagen wird zwar ein versteifter Bereich gebildet, der die Stützkraft aufnehmen kann, jedoch ist wiederum der Tragekomfort erheblich verringert.

Auch aus der EP 1 379 201 B1 ist eine ähnliche, weitere Fußgelenksorthese bzw. eine Knöchel-Fuß-Orthese bekannt geworden. Aus der US -A-6 146 344 ist eine Fußhebeorthese bekannt, bei der ein aus Kohlefasern bestehende tubuläres multidirektionales Geflecht Verwendung findet. Aus der US-A-5 826 304 ist eine Flexur-Einheit einer Gelenkverbindung mit Karbonfasern bekannt, welche der Einheit eine longitudinale Festigkeit und Steifheit verleihen.

Der Erfindung liegt die Aufgabe zugrunde, eine Orthese und ein Verfahren zu deren Herstellung vorzuschlagen, welche eine individuelle Anpassung an die Belastung sowie einen hohen Tragekomfort mit einer hinreichenden Steifigkeit und dynamischen Belastbarkeit ermöglicht

Diese Aufgabe wird erfindungsgemäß durch eine Orthese gemäß den Merkmalen des Anspruchs 1 gelöst. Weitere vorteilhafte Ausführungsformen und Weiterbildungen sind in den weiteren Ansprüchen angegeben.

Die erfindungsgemäße Orthese umfasst einen Stützkörper mit einem ersten und zumindest einem weiteren Stützabschnitt aus zumindest einem Verstärkungsgebilde, welches eine Trägerlage umfasst, auf welcher ein endlos gelegtes Bündel von Verstärkungsfasern aufgebracht, insbesondere mittels eines Befestigungsfadens aufgenäht oder aufgestickt ist, wobei eine Legung der Faserbündel weitgehend entsprechend den im Stützkörper auftretenden Spannungen kraftlinienorientiert vorgesehen ist. Die Legung der Faserbündel erfolgt ferner im Wesentlichen innerhalb einer Fläche auf der Trägerlage, die einer Flächenabwicklung des Stützkörpers entspricht und die mit den aufgebrachten Faserbündeln räumlich verformt und mit einer aushärtbaren Grundmasse getränkt wird und den Stützkörper bildet.

Unter einem endlos gelegten Bündel von Verstärkungsfasern wird ein Faserbür del verstanden, das entlang einer vorgegebenen Linie, die im Wesentlichen innerhalb der Fläche der Trägerlage, die einer Flächenabwicklung des Stützkörpers entspricht, unterbrechungsfrei verlegt wird. Es werden also keine kurzen, voneinander getrennte Bündelabschnitte, sondern ein "unendlich" langes Bündel von Verstärkungsfasern verarbeitet. Dabei ist nicht ausgeschlossen, dass nicht nur ein einziges Faserbündel im Wesentlichen innerhalb des Stützkörpers verlegt wird, sondern es können auch zwei oder mehrere Faserbündel entsprechend verlegt sein. Die Anzahl der mehreren Faserbündel ist dann aber vorzugsweise gering, so dass ein dem einen Faserbündel entsprechender technischer Effekt erzielt wird. Je geringer die Anzahl der Faserbündel ist, desto geringer ist auch der in der Fertigung auftretende Beschnitt an Kohlenstofffasern. Ein geringerer Beschnitt führt zu einer geringeren Bildung von Kohlenstoffstaub und somit zu einer geringeren Brandgefahr.

Die vorgegebene Linie, entlang derer das Faserbündel verlegt wird, verläuft entsprechend den im Stützkörper auftretenden Spannungen weitgehend kraftlinienorientiert. Die vorgegebene Linie kann dabei im Wesentlichen geradlinig verlaufend Abschnitte entlang den auftretenden Spannungen sowie sich daran anschließende 180° Umlenkungen aufweisen, so dass ein Verlegen des Faserbündels entlang der kraftlinienorientierten Richtung hin und zurück erfolgt. Die Länge des Faserbündels kann dabei, je nach zu realisierender Orthese, in einem bevorzugten Bereich von 20 Metern bis wenigen hundert Metern und vorzugswiese bei Knöchel-Fuß-Orthese im Bereich von 40 bis 80 Metern liegen. Ein solcher Stützkörper für eine Orthese weist den Vorteil auf, dass aufgrund der kraftlinienorientierten Ausrichtung des Faserbündels mit Verstärkungsfasern auf der Trägerlage sowohl eine erhöhte Steifigkeit als auch eine erhöhte Flexibilität bzw. dynamische Belastbarkeit bei einer verringerten Dicke des Verstärkungsgebildes erreicht werden kann.

Zur Bildung des Stützkörpers wird ein Verstärkungsgebilde hergestellt, bei dem die Faserbündel innerhalb der Fläche auf der Trägerlage insbesondere aufgenäht oder aufgestickt werden, die einer Flächenabwicklung des Stützkörpers entspricht. Vorzugsweise wird der Kraftfluss der Faserbündel nicht unterbrochen. Ein Verlegen der Faserbündel insbesondere aus der Fläche heraus und nach Umlenkung außerhalb dieser Fläche wieder in die Fläche hinein ist unerwünscht und soll nicht erfolgen bzw. gegebenenfalls mit der Ausnahme des Verlegens eines Zuführfaserbündels, der aus legetechnischen Gründen von außerhalb der Fläche zugeführt wird. Aufgrund der durchgängigen Ausbildung des Bündels von Verstärkungsfasern wird eine erhöhte Kraftübertragung ermöglicht, wodurch bei einer geringen Dicke des Verstärkungsgebildes bereits eine gleiche oder erhöhte Steifigkeit im Vergleich zu gängigen Orthesen erzielt werden kann. Durch den Verlegeverlauf der Faserbündel innerhalb der Fläche der Trägerlage, die der Flächenabwicklung des Stützkörpers entspricht, erfolgt auch eine Umlenkung bei der Verlegerichtung der Faserbündel vorzugsweise ausschließlich innerhalb der Flächenabwicklung, so dass eine Unterbrechung der Faserbündel nicht vorgesehen ist.

Vorzugsweise erfolgt die Legung des vorzugsweise einen Bündels mit Verstärkungsfasern derart, dass die Fläche der Trägerlage, die einer Flächenabwicklung des Stützkörpers entspricht, im Wesentlichen wenigstens weitgehend und vorzugsweise vollständig von Verstärkungsfasern abdeckt wird. Die Verstärkungsfasern sind dabei vorzugsweise die Fasern des endlos verlegten Bündels.

Die Trägerlage kann dabei aus einem Kunststoff, beispielsweise einem Polyamidgewebe oder -vlies gebildet sein.

Allerdings ist auch denkbar, dass die Trägerlage aus einem faserverstärkten Vlies oder Gewebe, insbesondere einem kohle- oder glasfaserverstärkten Vlies oder Gewebe, besteht oder dass die Trägerlage eine aus Verstärkungsfasern bestehende Grundgelegeschicht ist. Insbesondere kann die Trägerlage auch von einem Kreuzgelege aus Verstärkungsfasern, die den Verstärkungsfasern des Bündels ähnlich oder identisch sind, bestehen. Bei einer solchen Ausführungsform ist dann ausreichend, wenn die Grundgelegeschicht zusammen mit dem entlang den im Stützkörper auftretenden Spannungen kraftliniennorientiert verlegten Bündel die Fläche der Trägerlage, die der Flächenabwicklung des Stützkörpers entspricht, im Wesentlichen wenigstens weitgehend und vorzugsweise vollständig abdeckt.

Vorzugsweise ist vorgesehen, dass die Aufbringung des Faserbündels mittels eines Befestigungsfadens durch Aufnähen oder Aufsticken oder mittels eines Klebstoffes durch Aufkleben erfolgt. Ferner ist vorteilhaft, wenn das Bündel von Verstärkungsfasern von 3.000 bis 56.000 und vorzugsweise von 12.000 bis 48.000 und weiter vorzugsweise von 24.000 bis 36.000 parallel zueinander verlaufenden Fasern, insbesondere Karbonfasern, gebildet wird.

Nach einer bevorzugten Ausgestaltung der Erfindung ist zumindest eine Gelegeschicht auf der Trägerlage vorgesehen, welche von den endlos gelegten Faserbündeln gebildet ist. Diese Ausgestaltung ermöglicht eine kontinuierliche Verlegung und insbesondere Vernähung des Faserbündels zur Trägerlage sowie eine für den Kraftfluss geschlossene Fläche.

Bei einer weiteren bevorzugten Ausgestaltung des Verstärkungsgebildes zur Herstellung des Stützkörpers ist vorgesehen, dass entlang des Randes und/oder parallel zum Rand der Fläche der Trägerlage, die der Flächenabwicklung des Stützkörpers entspricht, ein Bündel von Verstärkungsfasern als Randfaserbündel, insbesondere geschlossenes Randfaserbündel, gelegt ist. Diese Anordnung ermöglicht insbesondere eine Verstärkung bzw. Versteifung des Verstärkungsgebildes nach dessen Laminierung mit einer aushärtbaren Grundmasse. Dadurch ist gleichzeitig auch eine Bremse für die Legung des oder der weiteren Faserbündel von einer oder mehreren Gelegeschichten auf der Trägerlage gegeben. Dadurch kann auch zwischen einem solchen Randfaserbündel und den innerhalb gelegten Abschnitten des Faserbündels eine Verbindung erzielt werden. Der Randbereich bzw. das Randfaserbündel kann eine Breite im Bereich von 0,3 cm bis 3,5 cm und vorzugsweise im Bereich von 1 cm bis 2 cm aufweisen. Bei einer späteren Anpassung der Orthese an die die Orthese tragende Person kann im Randbereich Material entfernt werden, ohne dass dabei die mechanischen Eigenschaften oder deren Lebensdauer nachteilig verändert werden.

Eine weitere bevorzugte Ausgestaltung der Erfindung sieht vor, dass innerhalb einer Gelegeschicht die das Faserbündel unidirektional und/oder kreuzweise zueinander angeordnet verläuft. Unter unidirektional wird insbesondere eine Legung im Wesentlichen entlang einer Richtung hin und zurück und im Wesentlichen parallel zueinander und/oder parallel zum einem Randbereich verstanden. Diese Auswahl für die Legung des Faserbündels steht in Abhängigkeit des jeweiligen Bereiches der Orthese und deren Funktion, so dass die Festigkeit, Biegesteifigkeit und Torsionsfestigkeit als auch eine dynamische Belastbarkeit der einzelnen Abschnitte entsprechend eingestellt werden kann.

Eine weitere bevorzugte Ausgestaltung der Erfindung sieht vor, dass zwischen den zumindest zwei Stützabschnitten zumindest ein Verbindungsabschnitt ausgebildet ist und die zumindest zwei Stützabschnitte durch Faserbündel entlang des Verbindungsabschnittes miteinander verbunden sind, die vorzugsweise räumlich miteinander verformt sind. Dadurch kann eine dynamische Belastbarkeit zwischen räumlich unterschiedlich ausgerichteten Stützabschnitten bestimmt werden. Gleichzeitig wird eine einfache Herstellung durch einen Endlosfaden ermöglicht.

Eine weitere bevorzugte Ausgestaltung der Orthese sieht vor, dass die eine Gelegeschicht sich zumindest teilweise im Stützabschnitt und/oder Verbindungsabschnitt und/oder im Übergangsbereich zwischen dem zumindest einen Stützabschnitt und dem Verbindungsabschnitt erstreckt. Diese Anordnung ermöglicht eine individuell angepasste Ausgestaltung der Orthese. Beispielsweise kann dadurch in einem Stützabschnitt, wie beispielsweise entlang einer Fußsohle, eine unterschiedliche Steifigkeit eingestellt werden, um die Fußsohle einer natürlichen Abrollbewegung des Fußes anzupassen. Insbesondere kann ein Fersenbereich steifer und ein Ballenbereich flexibler ausgebildet werden. Dies kann durch die Größe der Fläche der einzelnen Gelegeschicht erzielt werden. Solche un-terschiedlichen Steifigkeiten können auch durch die flächenmäßige Anzahl der der Faserbündel erzielt werden. Beispielsweise kann sich eine Gelegeschicht über die gesamte Fläche des Verstärkungs-gebildes erstrecken, jedoch kann in einem Bereich eine geringe Anzahl von Bahnen der Faserbündel und in einem anderen Bereich eine erhöhte Anzahl der Bahnen von Faserbündeln ausgebildet werden, wodurch ebenfalls unterschiedliche Steifigkeiten oder Flexibilitäten ein-gestellt werden können. Zudem ist denkbar, dass in einem Randbereich, in dem eine besondere Verstärkung vorgesehen sein soll, beispielsweise in dem Bereich, in dem die Sohle beim Laufvorgang zuerst Bodenkontakt hat, eine besonders große Dichte von Faserbündeln realisiert wird.

Das Verstärkungsgebilde für den Stützkörper kann vorteilhafterweise eine erste Gelegeschicht aufweisen, bei der eine Verlegung des Faserbündel nur unidirektional ausgerichtet ist, so dass dadurch eine Grundgelegeschicht gebildet ist. Dadurch kann eine Grundfestigkeit und grundsätzliche Übertragung von Kräften erzielt werden, insbesondere kann dann die bevorzugt als oberflächliche Lage ausgebildete Gelegeschicht für eine vektoriell orientierte Festigkeit sorgen. Auf eine solche Grundgelegeschicht kann dann das endlos verlegte Faserbündel kraftlinienorientiert verlegt werden.

Das Verstärkungsgebilde für den Stützkörper kann des Weiteren zumindest eine Gelegeschicht bzw. Grundgelegeschicht umfassen, bei der die Legung der Verstärkung des Faserbündels kreuzweise erfolgt und insbesondere der Verlauf des Fadens diagonal zum Kraftlinienverlauf ausgerichtet ist. Dadurch wird eine sogenannte Torsionsgelegeschicht gebildet. Eine solche Torsionsgelegeschicht kann als Grundgelegeschicht vorgesehen sein oder als zweite Gelegeschicht auf eine Grundgelegeschicht aufgebracht werden oder als Zwischenschicht zwischen beispielsweise zwei Grundgelegeschichten eingebracht sein. Auf einer derartigen Schicht kann dann das endlos verlegte Faserbündel kraftinnenorientiert verlegt werden.

Zur Auslegung der Belastbarkeit einzelner Zonen oder Bereiche des Stützkörpers, insbesondere in Übergangsbereichen zwischen Stützabschnitten oder zwischen Stützabschnitten und einem Verbindungsab-schnitt, bei denen auch ein Übergang von einer Großfläche in einen ver-jüngten Bereich oder eine verkleinerte Fläche erfolgt, ist bevorzugt vorgesehen, dass mehrere Legungen der Faserbündel näher oder eng zueinander beabstandet, aneinander grenzend, teilweise überlappend und/oder überdeckend vorgesehen sind. Dadurch kann zumindest partiell die Biegefähigkeit, Torsionssteifigkeit und/oder Nachgiebigkeit in einzelnen Bereichen im Verstärkungsgebilde und somit im Stützkörper eingestellt werden. Ergänzend kann auch eine kreuzweise Legung der Faserbündel vorgesehen sein, so dass beispielsweise mehrere nebeneinander oder übereinander unidirektional verlaufende Abschnitte des oder der Faserbündel zueinander fixiert werden.

Bevorzugtsind zwischen einem ersten und einem weiteren Stützabschnitt ein verjüngter bzw, ein flächenmäßig reduzierter Verbindungsabschnitt und eine erste Gelegeschicht mit unidirektionaler Legung des Faserbündels vorgesehen, bei der im Übergangsbereich zwischen dem Stützabschnitt und dem verjüngten Abschnitt die Faserbündel eng aneinander liegend oder auch teilweise überlappend zueinander oder übereinander liegend angeordnet sind. Diese Anordnung ermöglicht einen fließenden Übergang an Engstellen, Verjüngungen oder Querschnittserweiterungen wie beispielsweise im Übergangsbereich zwischen zwei Stützabschnitten oder einem Stützabschnitt und einem Verbindungsabschnitt. Dadurch kann die Kerbwirkung und Rissbildung bei einer auftretenden Belastung erheblich reduziert werden.

Als Verstärkungsfasern können beispielsweise Filamentfasern aus Karbon, Glas, Aramid oder dergleichen Verwendung finden.

Bevorzugt ist die Orthese als Knöchel-Fuß-Orthese ausgebildet, bei welcher der erste Stützabschnitt zumindest teilweise als Fußsohle ausgebildet ist, der zumindest eine weitere Stützabschnitt als Unterschenkelanlage und ein Verbindungsabschnitt als Bügel zwischen der Fußsohle und der Unterschenkelanlage sich erstreckt. Bei einer solchen Knöchel-Fuß-Orthese ist die Ausgestaltung des Stützkörpers mit dem Verstärkungsgebilde besonders von Vorteil, da der Bügel dünn ausgebildet werden kann, so dass ein hoher Tragekomfort und eine sehr geringe Aufweitung des Schuhes gegeben ist. Darüber hinaus kann die erforderliche dynami-sche Belastung eingestellt und gleichzeitig eine hinreichende Kraftüber-tragung erzielt werden. Durch den kraftlinienorientierten Verlauf des Faserbündels wird darüber hinaus im Übergangsbereich zwischen dem Bügel und der Fußsohle, in dem eine große Querschnittsänderung als auch Kraftrichtungsänderung erfolgt, eine Rissbildung oder Kerbwirkung selbst bei sehr geringen, an die Anatomie des menschlichen Fußes angepassten Radien, verringert werden. Bei der Ausgestaltung des Stützkörpers zum Einsatz als Knöchel-Fuß-Orthese ist bevorzugt vorgesehen, dass der Bügel medial oder lateral verlaufend in die Fußsohle übergeht und vor, über oder hinter dem Fußknöchel verlaufend zwischen der Fußsohle und der Unterschenkelanlage angeordnet ist. Die Verwendung des Verstärkungsgebildes aus einer Trägerlage und mit Verstärkungsfasern ermöglicht eine flexible Ausgestaltung unter gleichbleibenden Herstellungsbedingungen für den Stützkörper. Dadurch kann bei gleichem Tragekomfort eine individuelle Anpassung der Orthese an den Patienten erfolgen.

Die der Erfindung zugrundeliegende Aufgabe wird des Weiteren durch ein Verfahren zur Herstellung einer Orthese gelöst mit den Merkmalen des Anspruchs 15 gelöst. Dabei kann insbesondere vorgesehen sind, dass ein Verstärkungsgebilde für einen Stützkörper hergestellt wird, in dem auf eine Trägerlage einendlos gelegtes Bündel von Verstärkungsfasern aufgebracht, insbesondere aufgenäht oder aufgestickt wird, wobei die Faserbündel wenigstens weitgehend entsprechend den im Stützkörper auftretenden Spannungen kraftlinienorientiert gelegt werden und die Legung der Faserbündel im Wesentlichen innerhalb einer Fläche auf der Trägerlage durchgeführt wird, die einer Flächenabwicklung des Stützkörpers entspricht. Anschließend wird das Verstärkungsgebilde, bestehend aus der Trägerlage und den darauf aufgenähten Faserbündel räumlich verformt und mit einer aushärtbaren Grundmasse getränkt, so dass ein Stützkörper ausgebildet ist.

Vorzugsweise ist vorgesehen, dass das Bündel der Verstärkungsfasern auf die Trägerlage aufgelegt und beim oder unmittelbar nach dem Auflegen auf der Trägerlage aufgenäht oder aufgestickt wird. Dazu kann eine Aufstick- oder Nähmaschine Verwendung finden, der das Endlosbündel beim Stick- oder Nähvorgang zugeführt wird.

Das beschriebene Herstellungsverfahren ermöglicht nicht nur eine individuelle Anpassung der Form des Stützkörpers an die Körperkontur des Patienten, sondern auch eine individuelle Einstellung bezüglich der dynamischen Belastungen abhängig von Körpergeometrie und dem Körpergewicht des Patienten. Somit wird durch dieses Verfahren die Herstellung einer individuellen Orthese in einfacher Weise ermöglicht. Gleichzeitig kann durch die kraftlinienorientierte Verlegung der Faserbündel ein in der Dicke gering aufbauender Stützkörper mit einer erforderlichen Steifigkeit und Flexibilität erzielt werden. Des Weiteren ermöglicht ein solches Verfahren eine kostengünstige Herstellung des Verstärkungsgebildes, da ausschließlich in der Fläche der Trägerlage Faserbündel aufge-bracht bzw. Gelegeschichten gebildet werden, die nachfolgend zur Bildung des Stützkörpers benötigt werden. Bei bisherigen Verfahren, bei denen zur Bildung eines Verstärkungsgebildes mehrere Gewebe,lagen übereinander gelegt werden, ist es erforderlich, dass einzelne Lagen aus einem bahnförmigen Material oder aus einzelnen Bögen von diesem Gewebe ausgeschnitten werden, wodurch ein erheblicher Abfall entsteht. Insbesondere beim Einsatz von Kohlefasergewebe wird durch dieses neue Verfahren zur Herstellung einer Orthese eine erhebliche Kosteneinsparung erzielt. Darüber hinaus entsteht weniger Beschnitt, wodurch weniger Kohlefaserstaub in die Umgebung gelangt. Dies führt zu einer erheblichen Reduzierung der Brandgefahr bei der Fertigung.

Eine bevorzugte Ausgestaltung des Verfahrens sieht vor, dass auf die Trägerlage zumindest eine Gelegeschicht insbesondere aufgenäht oder aufgestickt und anschließend die Trägerlage auf die Fläche zugeschnitten wird, welche der Flächenabwicklung des Stützkörpers entspricht. Dadurch wird die weitere Handhabung erleichtert. Insbesondere die Nacharbeit nach dem Laminieren der Trägerlage mit den Faserbündeln wird deutlich reduziert. Durch das zuschneiden der Trägerlagen besteht das Verstärkungsgebilde aus der Flächenabwicklung des herzustellenden Stützkörpers, so dass anschließend lediglich noch eine Nacharbeit zur Verrundung von Kanten oder Überständen oder dergleichen erforderlich ist, ohne die komplette Kontur des Stützkörpers zu überarbeiten.

Des Weiteren ist bevorzugt vorgesehen, dass für jede Gelegeschicht ein Bündel von Verstärkungsfasern endlos vernäht wird. Dadurch kann eine einfache und schnelle Herstellung sowie ein optimaler Kraftfluss innerhalb einer Gelegeschicht erzielt werden. Des Weiteren ist bevorzugt vorgesehen, dass mehrere Gelegeschichten auf derselben Trägerlage aufgenäht und aufgestickt werden. Dadurch kann ermöglicht werden, dass dem Grunde nach die Faserbündel untereinander bei mehreren Gelegeschichten miteinander verbunden werden und die Tragstruktur bilden und die Trägerschicht lediglich als Kopplungselement zwischen den einzelnen Faserbündeln bis zu deren Einlaminierung in eine aushärtbare Grundmasse dient.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
Figur 1 eine perspektivische Ansicht einer erfndungsgemäßen Orthese,
Figur 2 eine schematische Seitenansicht der Orthese gemäß Figur 1,
Figuren 3a - 3c und 4 einzelne Verfahrensschritte zur Herstellung der Orthese gemäß Figur 1,
Figur 5 eine schematische Ansicht von vorne auf eine alternative Ausführungsform der Orthese gemäß Figur 1,
Figur 6 eine schematische Seitenansicht der Orthese gemäß Figur 5,
Figuren 7a - 7c verschiedene schematische Ansichten zur Herstellung eines Verstärkungsgebildes für die Orthese gemäß Figur 5,
Figur 8 eine schematische Ansicht von vorne für eine weitere alternative Ausführungsform der Orthese und
Figur 9 eine perspektivische Ansicht auf eine Hüftspange einer Hüftorthese.

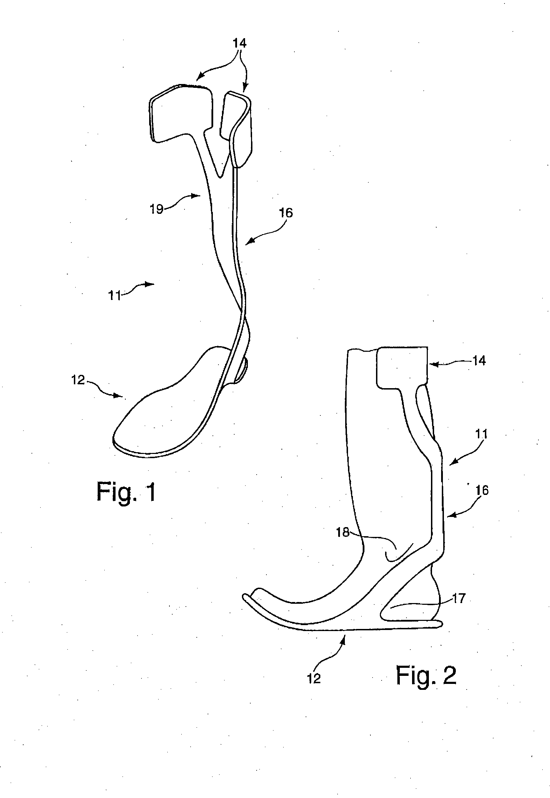

In Figur 1 ist perspektivisch eine erste Ausführungsform einer Orthese 11 dargestellt. Bei dieser Orthese 11 handelt es sich beispielsweise um eine Knöchel-Fuß-Orthese, welche bei Patienten mit hängendem Fuß oder Muskelschwäche in den unteren Extremitäten eingesetzt wird. Diese Orthese 11 umfasst einen ersten Stützabschnitt 12, der beispielsweise als Fußsohle ausgebildet ist. Des Weiteren umfasst diese Orthese 11 zumindest einen zweiten Stützabschnitt 14, der als Unterschenkelanlage ausgebildet ist. Bei dieser Orthese 11 ist der zweite Stützabschnitt 14 als Wadenanlage vorgesehen. Zwischen dem ersten und zweiten Stützabschnitt 12, 14 ist ein Verbindungsabschnitt 16 vorgesehen, der als Bügel ausgebildet ist und einteilig von dem Stützabschnitt 12 in den Stützab-schnitt 14 übergeht. Der Stützabschnitt 14 umgreift die Wade zumindest teilweise. Ergänzend sind an den vorderen freien Enden der Stützabschnitte 14 nicht näher dargestellte Befestigungsmittel, wie beispielsweise Bänder mit Klettverschlüssen oder dergleichen, befestigt, um den Stützabschnitt 14 am Unterschenkel zu fixieren. Eine solche Knöchel-Fuß-Orthese wird auch als Unterschenkelorthese bezeichnet.

Aus Figur 2, einer Seitenansicht der Orthese 11 gemäß Figur 1, ist zu ersehen, dass der Stützabschnitt 12 bevorzugt einen annähernd ana-tomischen Fußsohlenverlauf umfasst. Darüber hinaus ist zwischen dem ersten Stützabschnitt 12 und dem Verbindungsabschnitt 16 ein Übergangsbereich 17 geschaffen, der sich ausgehend von der Fußsohle stegförmig verjüngt und im Wesentlichen gegenüber der horizontalen Ausrichtung der Fußsohle in vertikaler Richtung nach oben ausgerichtet ist und in den Verbindungsabschnitt 16 übergeht, der sich in einem ersten Abschnitt hinter dem Knöchel zur Achillessehne und Wade erstreckt und in einem zweiten Abschnitt entlang der Wade nach oben geführt ist, bevor dieser in den oberen Stützabschnitt 14 übergeht.

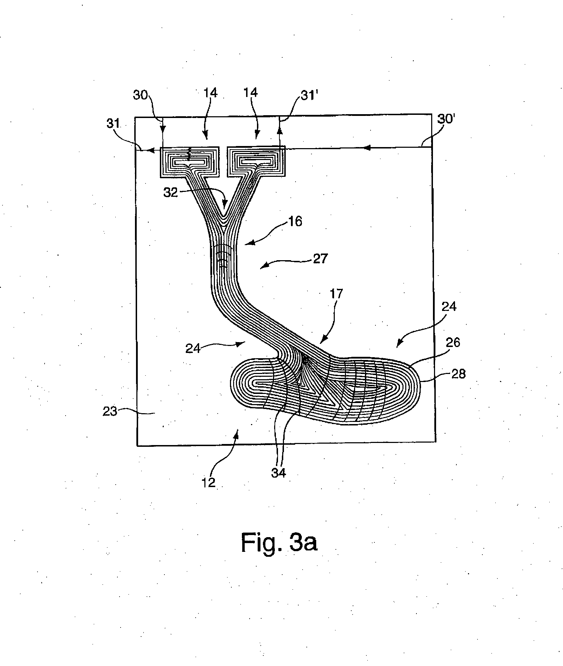

Diese in den Figuren 1 und 2 dargestellte Orthese 11 ist aus einem Stützkörper 19 ausgebildet, der die Stützabschnitte 12 und 14 und den Verbindungsabschnitt 16 umfasst. Dieser Stützkörper 19 besteht aus einem Verstärkungsgebilde 21, dessen Aufbau in den Figuren 3a bis 3d näher dargestellt und dazu beschrieben wird. Das Verstärkungsgebilde 21 umfasst eine Trägerlage 23, die aus einem faserverstärkten Vlies oder Gewebe, insbesondere einem Polyamidvlies oder einem kohle- oder glasfaserverstärkten Vlies oder Gewebe, besteht. Diese Trägerlage 23 kann als bahnförmiges Mate-rial oder Bogen bereitgestellt werden. Auf diese Trägerlage 23 wird zumindest eine Gelegeschicht 24 aufgebracht. Diese Gelegeschicht 24 wird gemäß einer ersten Ausführungsform aus einem endlos gelegten Bündel 26 von Verstärkungsfasern gebildet, das mittels eines Befestigungsfadens auf die Trägerlage 23 aufgenäht oder aufgestickt wird. Der Verlauf des Verstärkungsfaserbündels bzw. die Legung der Faserbündel 26 wird durch eine Fläche 27 auf der Trägerlage 23 bestimmt, die einer Abwicklung des dreidimensionalen Stützkörpers 19 in eine zweidimensionale Ebene entspricht. Vorzugsweise ausschließlich innerhalb dieser Fläche 27 wird das Bündel 26 mit Verstärkungsfasern gelegt. Bevorzugt ist dabei vorgesehen, dass zur Bildung des endlosen Bündels 26 Verstärkungsfasern innerhalb der Fläche 27 zur Bildung einer Gelegeschicht 26 gelegt werden. Das Bündel 26 kann dabei insbesondere aus 24.000 einzelnen, parallel verlaufenden Karbonfasern gebildet sein.

Bevorzugt wird zunächst eine durchgehendes äußeres Randfaserbündel 28 gelegt, das entlang dem Rand der Fläche 27 verläuft und vorzugsweise diese Fläche 27 vollständig einschließt. Im Anschluss daran können mehrere unidirektional ausgerichtete Legungen der Faserbündels 26 erfolgen, wie dies beispielsweise in Figur 3a dargestellt ist. Dabei wird die Ausrichtung des Faserbündels 26 bevorzugt kraftlinienorientiert entsprechend den auftretenden Spannungen in der Orthese 11 aus-gerichtet. Ebenso kann auch zunächst eine Legung des Faserbündels 26 innerhalb der Fläche 27 und zum Schluss das Randfaserbündel 28 gelegt werden. Ebenso ist möglich, dass für die Legung des Randfaserbündels 28 und der innerhalb angeordneten Bahnen des Bündels jeweils getrennte Faserbündel 26 verwendet werden.

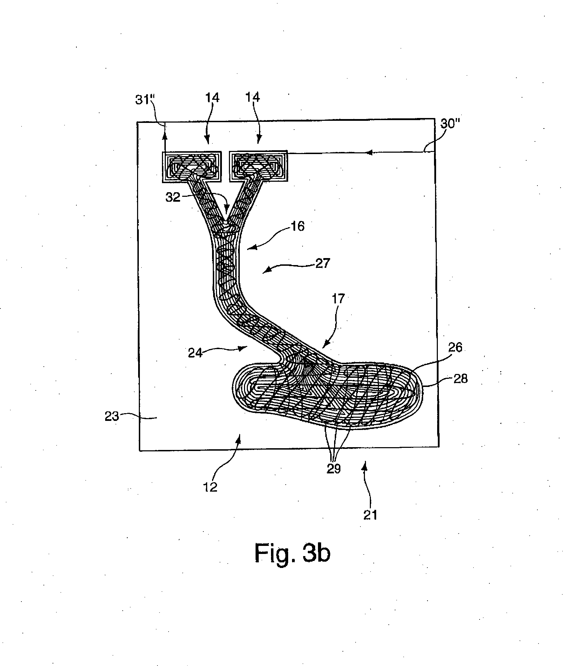

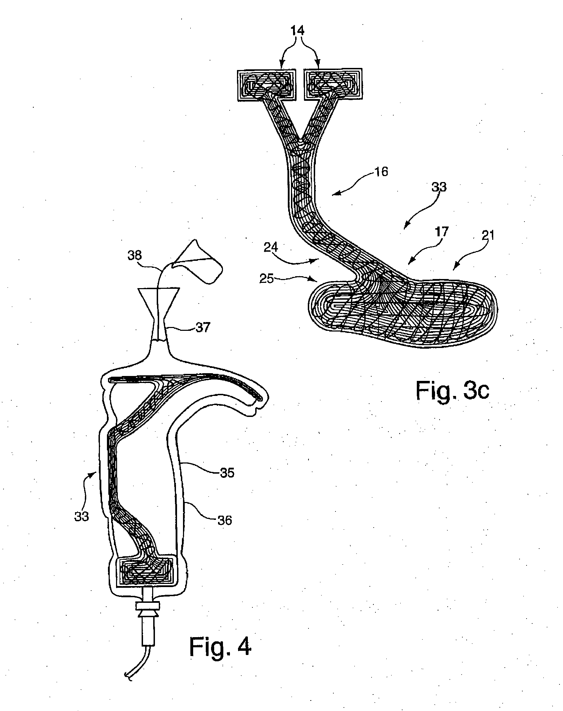

Bei dieser ersten Gelegeschicht 24 wird bevorzugt zunächst die gesamte Fläche 27 mit einem Bündel aus Verstärkungsfasern 26 bedeckt, wobei bevorzugt eine unidirektionale, das heißt eine im Wesentlichen entlang einer Richtung hin und zurück weitgehend parallel zueinander bzw. zur Randlinie verlaufenden Ausrichtung gewählt wird. Die Faserbündel 26 werden beispielsweise im ersten Stützabschnitt 12, der die Fußsohle bildet, mit größerem Abstand der einzelnen Bahnen zueinander gelegt, um zunächst eine Grundsteifigkeit zu erzielen. Damit eine hinreichende Festigkeit im Übergangsbereich 17 gegeben ist, werden in diesem Bereich die Bahnen der Faserbündel eng aneinander liegend, teilweise überlappend oder übereinander liegend durchgeführt. Dies weist zum Einen den Vorteil auf, dass mit durchgehenden Faserbündel für die erste Gelegeschicht gearbeitet werden kann, wodurch eine schnelle Verarbeitung ermöglicht wird, ohne dass Knoten an einer Anschlussstelle entstehen. Darüber hinaus kann ein kraftfluss-begünstigter Übergang geschaffen wird, der eine erhöhte Knickbelastung ermöglicht und für eine geringe Rissbildung sorgt. Alternativ kann auch vorgesehen sein, dass einzelne Abschnitte oder Bereiche sowohl in den Stützabschnitten 12 und 14 als auch im Verbindungsabschnitt 16 freibleibend oder ausgespart sind. Ebenso können einzelne Bereiche mit einer erheblich verringerten Anzahl an Schlaufen oder Bahnen von Faserbündeln 26 ausgebildet werden, wodurch die Steifigkeit der Stützabschnitte 12 und/oder 14 des Verbindungsabschnittes 16 bestimmt wird. Im Ausführungsbeispiel gemäß Figur 3a erfolgt die Verlegung des Randfaserbündels 28 über Zuführfaserbündel bzw. einen Zuführbündelabschnitt 30 und das Ende der Faserbündel 26 für die Randfaserbündel 28 wird über Abführfaserbündel bzw. einen Abführbündelabschnitt 31 herausgeführt und bevorzugt außerhalb der Fläche 27 oder der Trägerlage 23 abge-trennt. Die in Figur 3a dargestellte Gelegeschicht 24 bildet eine Grundgelegeschicht, durch welche eine gewisse Oberflächensteifigkeit der Orthese 11 zusammen mit der Trägerlage 23 ermöglicht wird. Des Weiteren kann bei der Ausführungsform gemäß Figur 3 vorgesehen sein, dass für die innerhalb der Randfaserbündel 28 liegenden Bahnen der Faserbündel 26 ein separater Zuführfaden 30' und Abführfaden 31 'vorgesehen ist. Ebenso können Faserbündel 26 verwendet werden, die die Randfaserbündel 28 bilden. Jegliche Umlenkung der Faserbündel 26 erfolgt vorzugsweise innerhalb der Fläche 27 bzw. innerhalb der Rand-faserbündel 28 oder an die Randfaserbündel 28 angrenzend bzw. diese überlappend, ohne jedoch deutlich außerhalb der Randfaserbündel 28 zu liegen. Durch diese bevorzugte Ausgestaltung des Verstärkungsgebildes 21 durch die Aufbringung des Bündels 26 von Verstärkungsfasern wird ermöglicht, dass Übergangsbereiche mit unterschiedlichen Flächengrößen, wie beispielsweise zwischen der Fußsohle und dem Bügel, kraft-flussorientiert ausgebildet werden können. Analog gilt dies für die Übergangsbereiche zwischen dem Verbindungsabschnitt 16 und den dargestellten Stützabschnitten 14.

Gemäß einer bevorzugten Ausführungsform ist eine Gabelung 32 nicht vorgesehen und ein Stützabschnitt 14, beispielsweise im Umfang der beiden dargestellten Stützabschnitte mit einem vollflächigen Verbindungsabschnitt 16 verbunden. Des Weiteren kann durch eine solche Legetechnik der Faserbündel 26 auch eine kraftflussorientierte Gabelung 32 des Verbindungsabschnittes 16 erzielt werden, so dass beide Äste der Gabelung 32 belastungsoptimiert an dem Verbindungsabschnitt 16 angebunden sind.

Die Gelegeschicht 24 wird auf der Trägerlage 23 derart aufgenäht oder aufgestickt, dass zu einem späteren Zeitpunkt, wie dies aus einem Übergang 3c zu Figur 3d hervorgeht, beispielsweise der Stützabschnitt 12 in einem größeren Winkelbereich, beispielsweise zwischen 70° und 110° zum Verbindungsabschnitt 16 verformt werden kann, um einen im We-sentlichen horizontal ausgerichteten Stützabschnitt 12 als Fußsohle und einem wesentlich vertikal ausgerichteten Verbindungsabschnitt 16 als Bügel auszubilden. Diese Verformbarkeit ist für diese Orthese 11 nur beispielhaft. In Abhängigkeit von weiteren Einsatzfällen von Orthesen können die Verformungen auch größere oder kleinere Winkelgrade aufweisen. Durch die auch kraftflussorientierte Ausrichtung der Bahnen der Faserbündel 26 wird eine solche Umlenkung begünstigt und gleichzeitig die gewünschte Steifigkeit bei einer verringerten Wandstärke aufrechterhalten.

Die Faserbündel 26 werden nach bekannten Techniken vernäht oder aufgestickt, wobei beispielsweise in Übergangsbereichen 17, die eine Querschnittsveränderung umfassen und/oder in räumlich verformten Bereichen, bei denen eine Richtungsänderung in der Erstreckungsebene gegeben ist, die einzelnen Abschnitte des Faserbündels 26 mit einem gewissen Spiel auf der Trägerlage 23 aufgenäht werden können, um diese Veränderung mitzugehen und sich zumindest geringfügig an diese räumliche Veränderung anzupassen. Zur einfache-ren Herstellung einer Gelegeschicht 24 mit endlosen Faserbündel 26 können ein oder mehrere Umlenkfadenabschnitte 34 vorgesehen sein, um verschiedene Bahnverläufe oder Strukturen von Bahnverläufen herzustellen und somit gegebenenfalls auch verschiedene Festigkeitsbereiche zu schaffen.

Zur Erhöhung der Steifigkeit des Verstärkungsgebildes 21 kann eine zweite Gelegeschicht 25 der ersten Gelegeschicht24 überlagert werden. Dies ist beispielsweise in Figur 3b dargestellt, indem die zweite Gelegeschicht 25 durch kreuzweise Legung der Faserbündels 26 erfolgt. Die Umlenkbereiche 29 für die kreuzweise Verlegung des Faserbündels 26 sind insbesondere an die Randfaser 28 angrenzend, auf oder innerhalb der Randfaser 28 vorgesehen. Durch eine solche kreuzweise Verlegung kann eine Torsionsschicht geschaffen werden. Anstelle einer solchen Torsionsschicht kann auch eine entsprechend der Form des Stützabschnitts ausgeschnittene Gewebeschicht, insbesondere ein Kreuzgelege aus Faserbündeln 26, Verwendung finden.

Für den weiteren Aufbau des Verstärkungsgebildes 21 können insbesonde-re im Übergangsbereich 17 zusätzliche Gelegeschichten eingebracht werden, die sich beispielsweise in den Verbindungsabschnitt 16 erstrecken und sich zumindest teilweise in den Stützabschnitt 12 hinein erstrecken, um den Übergangsbereich 17 zu verstärken. Des Weiteren kann durch die Auswahl der Legung der Faserbündel 26 im Stützabschnitt 12 eine unterschiedliche Flexibilität bzw. Steifigkeit der Fußsohle eingestellt werden, so dass beispielsweise ein Fersenbereich steifer und ein Fußbeinbereich weicher ausgebildet ist, um insbesondere bei hängenden Füßen einem Nachziehen des Fußes entgegenzuwirken und gleichzeitig durch das weichere Vliesteil ein angenehmere Auftreten zu ermöglichen.

Nachdem ein oder mehreren Gelegeschichten 24, 25 gemäß Figur 3b auf dieselbe Trägerlage 23 aufgenäht oder aufgestickt sind, wird die Träger-lage 23 bezüglich der Fläche 27 ausgeschnitten, so dass ein Zwischen-produkt 33 gemäß Figur 3c hergestellt ist. Alternativ kann des Weiteren vorgesehen sein, dass zwischen einzelnen Gelegeschichten eine weitere Trägerlage aufgebracht wird, um darauf eine weitere Gelegeschicht aufzunähen, so dass ein Mehrschichtaufbau aus Trägerlagen und Gelegeschichten anstelle dem in Figur 3b dargestellten Ausführungsbeispiel hergestellt wird, bei dem eine Trägerlage und mehrere Gelegeschichten vorgesehen sind. Wie erwähnt, kann auch eine Gelegeschicht von einem entsprechend ausgeschnittenen Gewebe, insbesondere einem Kreuzgelege aus Faserbündeln, gebildet sein.

In einem nächsten Arbeitsschritt wird ein solches Zwischenprodukt 33 zum Laminieren vorbereitet und auf ein Positivmodell 35 vorfixiert. Anschließend wird beispielsweise eine Umhüllung 36 übergestülpt, und über eine Öffnung 37 wird eine aushärtbare Grundmasse 38 eingefüllt. Bei dieser aushärtbaren Grundmasse handelt es sich beispielsweise um ein Acryl- oder Epoxidharz oder um einen Thermoplast oder dergleichen. Diese aushärtbare Grundmasse 38 ist entsprechend dem Material der Trägerlage 23 und der Faserbündel 26 ausgewählt. Nach dem Einfüllen der Grundmasse 28 in die Umhüllung 36 und dem vollständigen Tränken des Zwischenprodukts 33 wird ein Unterdruck angelegt. Es erfolgt ein Aushärten der Grundmasse 38. Anschließend wird die Umhüllung 36 von der Positivform 35 entfernt und der gebildete Stützkörper 19 von dem Positivmodell 35 entnommen. Anschließend werden die Kanten bzw. Randbereiche des Stützkörpers 19 nachbearbeitet und verbunden, wobei bei dieser Nachbearbeitung bevorzugt eine Beschädigung oder Durchtrennung des Randfaserbündels 28 nicht erfolgt. Dadurch kann der geschlossene Kraftflusslinienverlauf erhalten bleiben, wodurch eine Orthese 11 mit einer hohen Beständigkeit gegen Abnützung und Rissbildung und einem hohen Tragekomfort ermöglicht wird.

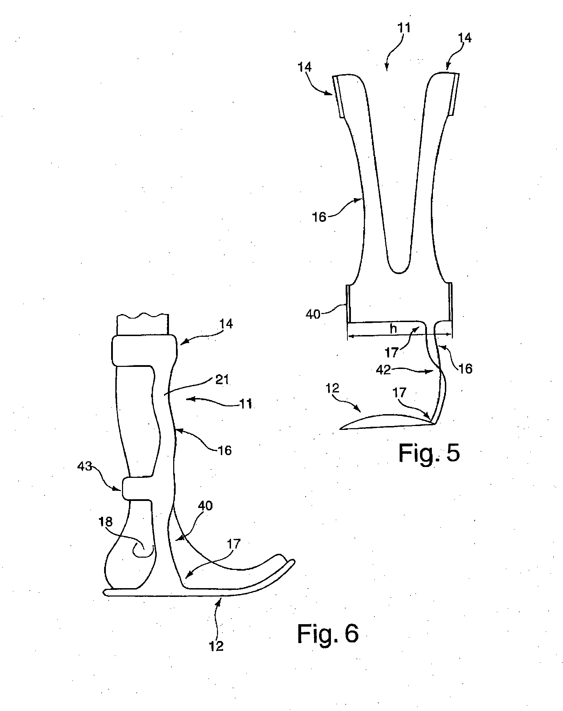

In den Figuren 5 und 6 ist eine alternative Ausführungsform einer Orthe-se 11 zu den Figuren 1 und 2 dargestellt. Der grundsätzliche Aufbau und die Funktion dieser Orthese 11 entspricht der gemäß dem ersten Ausfüh-rungsbeispiel. Diese Orthese 11 weicht beispielsweise dahingehend von der ersten Ausführungsform ab, dass der Verbindungsabschnitt 16 im unteren Bereich lateral und nicht medial zum ersten Stützabschnitt 12 angeordnet ist. Darüber hinaus ist dieser Verbindungsabschnitt 16 vor dem Fußknöchel 18 angeordnet. Alternativ kann dieser auch über dem oder hinter dem Fußknöchel 18 verlaufen. Im Ausführungsbeispiel sind zwei Verbindungsabschnitte 16 oberhalb des Knöchels dargestellt, die jeweils einen Stützabschnitt 14 tragen. Alternativ kann auch nur ein Verbindungsabschnitt 16 vor dem Schienbein nach oben geführt werden und einen Stützabschnitt 14 aufnehmen, der in der Größe bspw. den beiden dargestellten Stützabschnitten 14 entspricht. Ebenso kann der eine Verbindungsabschnitt 16 im Bereich des Schienbeins eine Aussparung aufweisen und seitlich zum Schienbein angeordnete Verbindungsabschnitte 16 umfassen, die in einen Stützabschnitt 14 münden. Insbesondere zeigt die Figur 6, dass eine bionische Bauform des Verbin-dungsabschnittes 17 im Bereich 40 vorgesehen ist. Aufgrund der Ausgestaltung des Verstärkungsgebildes 21 wird eine solche Verjüngung des Verbindungsabschnittes 17 im Knöchelbereich ermöglicht, ohne dass eine Beeinträchtigung in der dynamischen Belastbarkeit einer solchen Orthese 11 gegeben ist. Beispielsweise wird dieser vereng-te Bereich 40 durch ein entsprechendes enges Nebeneinanderlegen der Faserbündel 26 bzw. teilweise Überlappen oder Übereinanderlegen mit einer hinreichenden hohen Steifigkeit ermöglicht. Darüber hinaus wird durch die Herstellung des Stützkörpers 19 aus dem Verstärkungsgebilde 21 auch eine Verschränkung 42 im Verbindungsabschnitt 16 zwischen dem ersten und weiteren Stützabschnitt 12, 14 ermöglicht, wie dies aus Figur 5 hervorgeht. Dadurch kann eine noch bessere Anpassung des Verbindungsabschnitts 16 an den anatomischen Verlauf der unteren Extremität ermöglicht werden, wodurch ein Auftragen der Dicke der Orthese 11 weiter reduziert werden kann. Durch die bionische Bauform des Verbin-dungsabschnittes 16 im Bereich 40 und/oder der Verschränkung 42 kann sowohl mehr Platz für den Verbindungsabschnitt 16 innerhalb der Schuhöffnung bereitgestellt werden, als auch der druckerhöhende Kontakt zwischen dem Fußknöchel und dem Verbindungsabschnitt 16 verhindert werden. Gleichzeitig entsteht kein Verlust von mechanischen Eigenschaften am Verbindungsabschnitt.

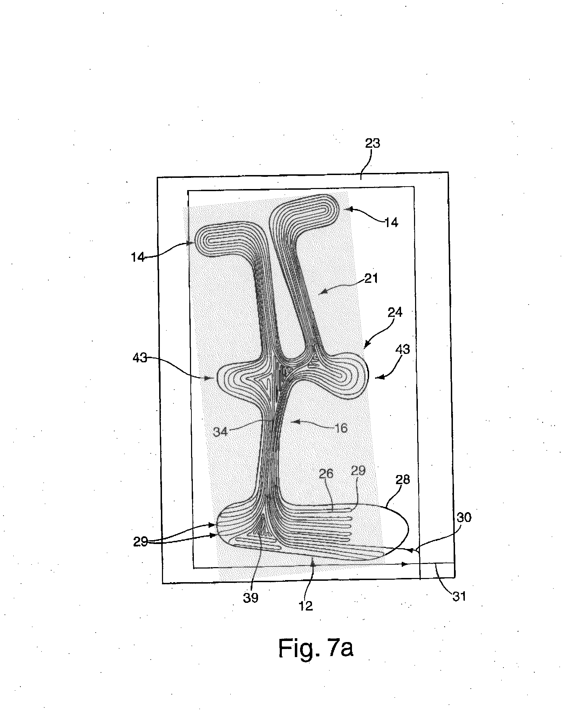

Des Weiteren kann bei einer solchen Orthese 11 in einfacher Weise eine Einbindung von Führungsanlagen 43, wie beispielsweise Pelotten, in den Stützkörper 19 eingebunden werden. Diese seitlichen Führungsanlagen dienen zur verbesserten Führung und Positionierung der Orthese 11 am Körper des Patienten. Darüber hinaus kann durch die Herstellung des Verstärkungsgebildes 21 und dessen anschließenden räumlichen Verformbarkeit zur Herstellung des Stützkörpers 19 auch ermöglicht werden, dass diese Führungsanlagen 43 angepasst und ausgerichtet werden. So kann beispielsweise ein unterer Randbereich der Führungsanlagen nicht formschlüssig an dem Fuß des Patienten anliegen, sondern mit einem geringen Abstand ausgerichtet sein. Dies weist den Vorteil auf, dass während der dynamischen Belastung der Orthese 11 in diesem Bereich keine Druckerhöhung auf die Körperoberfläche über die Orthese 11 erfolgt und dennoch ein anatomisch formschlüssiges Anliegen der Orthe-se 11 ermöglicht ist. Bei dieser Orthese 11 gemäß Figur 5 und 6 verläuft der Verbindungsabschnitt 16 vor und/oder seitlich zum Schienbein. Die Herstellung des Verstärkungsgebildes 21 über die Gelegetechnik ermöglicht wahlweise eine solche Ausgestaltung. Diese Ausführungsform der Orthese 11 besteht beispielsweise aus einem Verstärkungsgebilde 21 mit einer ersten Gelegeschicht 24 gemäß Figur 7a. Diese Gelegeschicht 24 weist beispielsweise ein endlos gelegtes Bündel von Verstärkungsfasern 26 auf, die sich bis zum Fußballen erstrecken, wobei der Zehenbereich ebenso weitgehend ausgespart ist. Darüber hinaus ist zu ersehen, dass im Übergangsbereich 17 die Faserbündel 26 enger zusammenlaufen und teilweise übereinander liegend in den Verbindungsabschnitt 16 übergeführt werden. In einem dadurch entstehenden Zwickel 39 gegenüberliegend zum Übergangsbereich 17 zwischen dem Verbindungsabschnitt 16 und dem Stützabschnitt 12 wird zur weiteren Aussteifung ebenso eine zwickelfüllende Legung der Faserbündel 26 eingebracht.

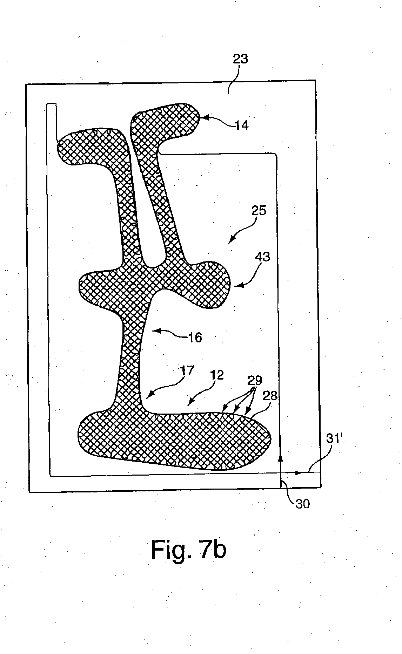

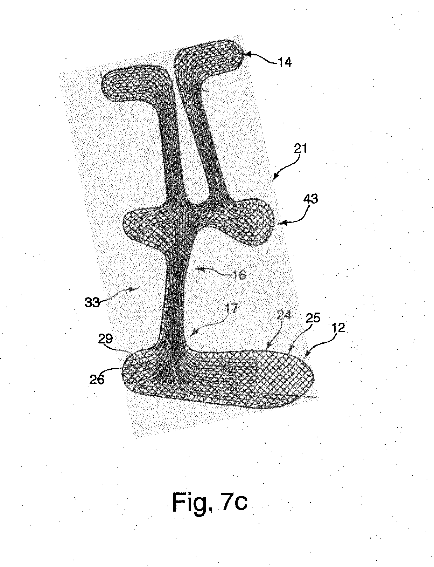

In Figur 7b ist beispielsweise eine weitere Gelegeschicht 25 dargestellt, die eine kreuzweise Legung umfasst. Die Umlenkungen 29 liegen alle auf oder innerhalb des Randfaserbündels 28 bzw. grenzen an diese an. Zur besseren Visualisierung ist diese Gelegeschicht 25 separat dargestellt. Bei einem Aufbau eines Stützkörpers 19 für die Orthese 11 gemäß den Figuren 5 und 6 wird beispielsweise zuerst die Gelegeschicht 24 auf die Trägerlage 23 aufgebracht und anschließend die Gelegeschicht 25 gemäß Figur 7b, oder umgekehrt. Ergänzend kann darüber nochmals eine Gelegeschicht 24 oder eine weitere Ausführungsform der Gelegeschicht aufgenäht oder gestickt werden, so dass sich daraus ein Zwischenprodukt 33 gemäß Figur 7c ergibt. Die weitere Fertigstellung des Stützkörpers 19 aus dem Zwischenprodukt 33 gemäß Figur 7c erfolgt in Analogie zu den beschriebenen Verfahrensschritten in Figur 3d.

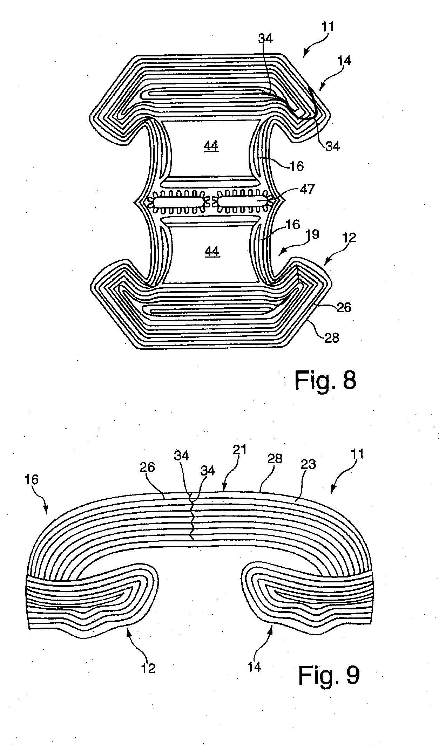

In Figur 8 ist eine weitere alternative Ausgestaltung einer Orthese 11 dargestellt. Bei dieser Orthese 11 handelt es sich beispielsweise um einen Überbrückungsrahmen für eine MLO Komfort-Orthese. Zwischen einem ersten und weiteren Stützabschnitt 12, 14 sind Verbindungsab-schnitte 16 vorgesehen, die jedoch durch Aussparung 44 zueinander beabstandet sind. Die Verbindungsabschnitte 16 umfassen einen Mittelsteg 45, der die Verbindungsabschnitte 16 zueinander aussteift. In dem Mittelsteg 45 sind weitere schlitzförmige Ausnehmungen 47 enthalten, die zur Aufnahme von nicht näher dargestellten Befestigungsmitteln dienen. Das Verstärkungsgebilde 21 zur Bildung des Stützkörpers 19 wird analog zu den vorgeschriebenen Ausführungsformen aufgebaut. Um die erforderlichen Stützkräfte zu übertragen, weist das Verstärkungsgebilde 21 die beispielhaft dargestellte Gelegeschicht 24 mit einer endlosen Legung des Bündels 26 von Verstärkungsfasern auf. Weitere Gelegeschichten 24, 25 können auch mit abweichenden Verläufen zur Legung des oder der Faserbündel 26 vorgesehen sein. Bevorzugt ist auf der Innenseite der Orthese 11 das Trägerlage 23 angeordnet, und auf der Außenseite ist zumindest die obere Gelegeschicht und ggf. eine darunter liegende Gelegeschicht sichtbar. Eine solche Anordnung kann auch für alle anderen Ausführungsbeispiele gelten.

Die vorstehend beschriebenen Ausführungsbeispiele zeigen, dass der Aufbau eines Stützkörpers 19 aus einem Verstärkungsgebilde 21, beste-hend aus einer Trägerlage 23 und einer oder mehreren Gelegeschichten 24, 25, mit endlos gelegten Bündeln 26 von Verstärkungsfasern für beliebige Orthesen 11 eingesetzt werden kann und durch diese Formgebung des Verstärkungsgebildes 21 eine spezifische Anpassung an die Anlagefläche am Körper und an die dynamischen Belastungen gegeben ist. Darüber hinaus kann durch einen solchen Aufbau einer Orthese 11 eine Verdickung in kritischen und hochbelastbaren Bereichen verringert werden. Dadurch wird der Tragekomfort erhöht. Gleichzeitig kann durch die Ausbildung eines Randfaserbündels 28 bzw, den Verlauf des Faserbündels 26 am Rand der Fläche 27 der Trägerlage 23 eine Rissbildung verringert werden.

In Figur 9 ist eine weitere alternative Ausgestaltung einer Orthese 11 teilweise dargestellt. Hierbei handelt es sich um eine Dyna-Cox-Hüftorthese, durch welche eine orthetische Versorgung eines Hüftgelen-kes ermöglicht ist. Diese Orthese 11 umfasst eine Hüftspange gemäß Figur 9 sowie nicht näher dargestellte Gelenke und Bandagen, die am Oberschenkel angreifen, um im Zusammenwirken eine Sicherung der knöchernen Struktur zur Verhinderung von Luxationen und einer Unter-stützung des Muskelaufbaus über den gesamten Versorgungszeitraum zu ermöglichen. Dabei ist ein Dreipunktprinzip vorgesehen. Die Orthesen-Komponenten sorgen durch die Kondylenanlage medial, die subtrochantäre Pelotte und den Gegenhalt der Hüftspange für ein solches Dreipunktprinzip und eine dynamische Sicherung des Gelenkkopfes in der Gelenkpfanne.

Diese Hüftspange umfasst zumindest einen ersten Stützabschnitt 12 und einen weiteren Stützabschnitt 14, die über zumindest einen Verbindungsabschnitt 16 miteinander verbunden sind. Beispielsweise können an dem ersten und weiteren Stützabschnitt 12, 14 Befestigungsmittel vorgesehen sein, um diese Hüftspange auf der Hüfte zu fixieren.

Die Hüftspange kann teilweise in Bandagen eingebunden sein. Die Her-stellung einer solchen Hüftspange erfolgt nach den Prinzipien der vorbe-schriebenen Ausführungsformen. In Figur 9 ist die Hüftspange mit bei-spielsweise einer Gelegeschicht 24, insbesondere als oberste Gelegeschicht, die aus einem endlos gelegten Faserbündel 26 besteht, dargestellt. Ebenso können mehrere Gelegeschichten 24, 25 als auch Gelegeschichten mit weiteren alternativen Verläufen der Faserbündel 26 vorgesehen sein. Zunächst wird ein Verstärkungsgebilde 21 mit einer Trägerlage 23 und einer oder mehreren Gelegeschichten 24, 25 hergestellt und als Zwi-schenprodukt 33 ausgebildet, um dieses anschließend auf dem Positiv-modell 35 mit der Grundmasse 38 zu tränken und nachzubearbeiten, um die Figur 9 dargestellte Endform zu erzielen. Die in den vorbeschriebe-nen Ausführungsbeispielen erwähnten Ausführungsformen und Alternati-ven können auch bei der Herstellung dieser Hüftspange vorgesehen sein.

## Patentansprüche

1. Orthese, mit einem ersten Stützabschnitt (12) und zumindest einem weiteren Stützabschnitt (14), die einen räumlich geformten Stützkörper (19) bilden, wobei die Stützabschnitte (12, 14) zur Anlage am Patienten ausgebildet und zur Stützung von Gliedmaßen oder Körperoberabschnitten am Patient ausgebildet sind,
**dadurch gekennzeichnet,**
- **dass** der Stützkörper (19) mit dem ersten und zumindest einem weiteren Stützabschnit: (12, 14) aus einem Verstärkungsgebilde (21) besteht, welches eine Trägerlage (23) umfasst, auf welcher ein endlos gelegtes Faserbündel (26), das aus parallel zueinander verlaufenden Verstärkungsfasern gebildet wird, aufgebracht ist,
- **dass** eine Legung des Faserbündels (26) mit parallel zueinander verlaufenden Verstärkungsfasern wenigstens weitgehend entsprechend den im Stützkörper (19) auftretenden Spannungen kraftlinienorientiert vorgesehen ist,
- wobei die Legung des Faserbündels (26) innerhalb einer Fläche (27) auf der Trägerlage (23) erfolgt, die einer Flächenabwicklung des räumlich geformten Stützkörpers (19) entspricht und
- **dass** das mit einer aushärtbaren Grundmasse getränkte und räumlich verformte Verstärkungsgebilde (21) den Stützkörper (19) bilden.

2. Orthese nach Anspruch 1, **dadurch gekennzeichnet, dass** die Fläche der Trägerlage, d e einer Flächenabwicklung des Stützkörpers entspricht, von dem Faserbündel (26) im Wesentlichen wenigstens weitgehend und vorzugsweise vollständig abdeckt wird.

3. Orthese nach Anspruch 1 oder 2, **dadurch gekennzeichnet, dass** die Aufbringung des Faserbündels (26) mittels eines Befestigungsfadens durch Aufnähen oder Aufsticken oder mittels eines Klebstoffes durch Aufkleben erfolgt.

4. Orthese nach Anspruch 1, 2 oder 3, **dadurch gekennzeichnet, dass** das Faserbündel (26) von 3.000 bis 56.000 und vorzugsweise von 12.000 bis 48.000 und weiter vorzugsweise von 24.000 bis 36.000 Fasern gebildet wird.

5. Orthese nach einem der vorherigen Ansprüche, **dadurch gekennzeichnet, dass** das Faserbündel (26) zumindest eine Gelegeschicht (24, 25) auf der Trägerlage (23) bildet, wobei das Faserbündel (26) innerhalb der Gelegeschicht (24, 25) unidirektional und/oder kreuzweise zueinander ausgerichtet ist.

6. Orthese nach einem der vorherigen Ansprüche, **dadurch gekennzeichnet, dass** entlang des Randes und/oder parallel zum Rand der Fläche (27) der Trägerlage (23), die der Flächenabwicklung des Stützkörpers (19) entspricht, das Faserbündel (26) als Randfaserbündel (28), insbesondere als geschlossenes Randfaserbündel (28), gelegt ist.

7. Orthese nach einem der vorherigen Ansprüche, **dadurch gekennzeichnet, dass** das Randfaserbündel (28) eine Breite im Bereich von 0,3 cm bis 3,5 cm und vorzugsweise im Bereich von 1 cm bis 2 cm aufweist.

8. Orthese nach einem der vorhergehenden Ansprüche, **dadurch gekennzeichnet, dass** zwischen dem ersten und zumindest einen weiteren Stützabschnitt (12, 14) zumindest ein Verbindungsabschnitt (16) vorgesehen ist und die Stütrabschnitte (12, 14) durch Faserbündel (26) miteinander verbunden sind, wobei zumindest eine Gelegeschicht (24, 25) sich zumindest teilweise im Stützabschnitt (12, 14) und/oder Verbindungsabschnitt (16) und/oder Übergangsbereich (17) zwischen dem Stützabschnitt (12,14) und Verbindungsabschnitt (16) erstreckt.

9. Orthese nach einem der vorhergehenden Ansprüche, dadurch gekennzeichr et, dass innerhalb einer Gelegeschicht eine Legung des oder der Faserbündel (26) vorgesehen ist, die unidirektional zueinander ausgerichtet sind und eine Grundgelegeschicht bilden.

10. Orthese nach einem der Ansprüche 1 bis 6, **dadurch gekennzeichnet, dass** innerhalb einer Gelegeschicht (25) eine Legung des Faserbündels (26) kreuzweise erfolgt und insbesondere der Verlauf des Faserbündels (26) diagonal zum Kraftlinienverlauf im Stützkörper (19) ausgerichtet ist, so dass eine Torsionsgelegeschicht gebildet wird.

11. Orthese nach einem der vorhergehenden Ansprüche, **dadurch gekennzeichnet, dass** zur Erhöhung einer partiellen Steifigkeit des Stützkörpers (19) mehrere Legungen des Faserbündels (26) eng zueinander beabstandet, aneinander grenzend, teilweise überlappend und/oder überdeckend vorgesehen sind.

12. Orthese nach einem der vorhergehenden Ansprüche, **dadurch gekennzeichnet, dass** zwischen dem ersten und dem zumindest einen weiteren Stützabschnitt (12,14) ein flächenmäßig verjüngter Verbindungsabschnitt (16) vorgesehen ist und eine erste Gelegeschicht (24) mit unidirektional ausgerichtetem Faserbündel (26) ausgebildet ist, die in dem verjüngten Verbindungsabschnitt (16) aus dem Stützabschnitt (12, 14) kommend ihren Abstand zunehmend verringern und im Verbindungsabschnitt (16) eng aneinander liegend, teilweise überlappend oder übereinander liegend angeordnet sind.

13. Orthese nach einem der vorhergehenden Ansprüche, **dadurch gekennzeichnet, dass** der Stützkörper (19) als Knöchel-Fuß-Orthese ausgebildet ist und der erste Stützabschnitt (12) zumindest eine Fußsohle umfasst, der zumindest eine weitere Stützabschnitt (14) eine Unterschenkelanlage aufweist und der zumindest eine Verbindungsabschnitt (16) sich als Bügel zwischen der Fußsohle und der Unterschenkelanlage erstreckt.

14. Orthese nach einem der vorherigen Ansprüche, **dadurch gekennzeichnet, dass** der als Bügel ausgebildete Verbindungsabschnitt eine Verteilung des Faserbündels (26) derart aufweist, dass sich an das menschliche Sprungelenk angepasste mechanische Eigenschaften des Verbindungsabschnitts ergeben.

15. Verfahren zur Herstellung einer Orthese mit einem ersten Stützabschnitt (12) und zumindest einem weiteren Stützabschnitt (14), die einen räumlich geformten Stützkörper (19) bilden, wobei die Stützabschnitte (12, 14) zur Anlage am Patienten und zur Stützung von Gliedmaßen oder Körperabschnitten an Patienten ausgebildet sind, **dadurch gekennzeichnet,**
- **dass** zur Herstellung eines Verstärkungsgebildes (21) für einen Stützkörper (19) auf eine Trägerlage (23) ein endlos gelegtes Bündel (26), das aus parallel zueinander vorlaufenden Verstärkungsfasern gebildet wird, aufgebracht wird,,
- **dass** das Faserbündel (26) mit parallel zueinander verlaufenden Verstärkungsfasern wenigstens abschnittsweise entsprechend den im Stützkörper (19) auftretenden Spannungen kraftlinienorientiert gelegt wird,
- wobei die Legung des Faserbündels (26) innerhalb einer Fläche (27) auf der Trägerlage (23) durchgeführt wird, die einer Flächenabwicklung des Stützkörpers (19) entspricht, und
- **dass** die Trägerlage (23) und das darauf aufgenähte Faserbündel (26) räumlich verformt und mit einer aushärtbaren Grundmasse (38) getränkt werden.

16. Verfahren nach Anspruch 15, **dadurch gekennzeichnet, dass** das Bündel (26) der Verstärkungsfasern auf die Trägerlage aufgelegt und beim oder unmittelbar na ch dem Auflegen auf der Trägerlage (23) aufgenäht oder aufgestickt wird.

17. Verfahren nach Anspruch 15 oder 16, **dadurch gekennzeichnet, dass** nach dem Fertigste len des Aufbringens des Faserbündels (26) auf die Trägerlage (23) die auf di Fläche (27) zugeschnitten wird, welche der Flächenabwicklung des Stützkörpers (19) entspricht und vorzugsweise anschließend auf einem Positivmodell (35) zum Laminieren mit der aushärtbaren Grundmasse (38) positioniert wird.

18. Verfahren nach Anspruch 15, 16 oder 17, **dadurch gekennzeichnet, dass** ein oder mehrere Gelegeschichten (24, 25) auf die Trägerlage aufgebracht werden.

## Claims

1. Orthosis with a first support section (12) and at least one other support section (14), which form a spatially shaped support body (19), the support sections (12, 14) being configured to be attached to a patient and to support limbs or upper body sections of a patient, **characterized in that** the support body (19) with the first and at least one other support section (12, 14) consists of a reinforcing structure (21) that includes a support layer (23) on which a continuous laid out fiber bundle (26) is applied, that is formed from reinforcing fibers running parallel to one another, **in that** a layout of the fiber bundle (26) having reinforcing fibers running parallel to one another is provided at least substantially corresponding to stresses occurring in the support body, oriented along lines of stress, wherein the layout of the fiber bundle (26) is carried out within a surface area (27) of the support layer (23), corresponding to a two-dimensional layout of the spatially shaped support body (19), and that the spatially shaped reinforcing structure impregnated with a curable base material forms the support body (19).

2. Orthosis according to claim 1, **characterized in that** surface area of the support surface, corresponding to a two-dimensional layout of the support body is substantially covered at least to a large extent and preferably completely by the fiber bundle.

3. Orthosis according to claim 1 or 2, **characterized in that** the application of the fiber bundle (26) carried out via sewing or stitching by a fastening thread is, or via adhesive bonding by an adhesive.

4. Orthosis according to claim 1, 2 or 3, **characterized in that** the fiber bundle (26) is formed of 3,000 to 56,000 fibers and preferably of 12,000 to 48,000 and more preferably of 24,000 to 36,000 fibers.

5. Orthosis according to one of the preceding claims, **characterized in that** the fiber bundle (26) forms at least one layout layer (24, 25) on the support layer (23), wherein the fiber bundle (26) is oriented within the layout layer (24, 25) unidirectional or cross-wise.

6. Orthosis according to one of the preceding claims, **characterized in that** along the edge and/or parallel to the edge of the surface area (27) of the support surface (23) corresponding to the two-dimensional layout of the support body (19) the fiber bundle (26) is laid out as an edge fiber bundle (28), particularly as a closed edge fiber bundle (28).

7. Orthosis according to one of the preceding claims, **characterized in that** the edge fiber bundle has a width in the range of 0.3 cm-3.5 cm and preferably in the range of 1 to 2 cm.

8. Orthosis according to one of the preceding claims, **characterized in that** between the first and at least one other support sections (12, 14) at least one connecting section (16) is provided and the support sections (12, 14) are connected to one another by fiber bundles (26), wherein at least one layout layer (24, 25) extends at least partly into the support section (12, 14) and/or connecting section (16) and/or transition region (17) between the support section (12, 14) and the connecting section (16).

9. Orthosis according to one of the preceding claims, **characterized in that** within a layout layer (25) a layout of the fiber bundle (26) or bundles is provided, which are oriented to one another in a unidirectional manner, and which form a base layout layer.

10. Orthosis according to one of claims 1 to 6, **characterized in that** within a layout layer (25) a layout of the fiber bundle (26) is carried out in a cross-wise manner and in particular the course of the fiber bundle (26) is diagonally oriented to the course of the lines of stress within the support body (19) such that a torsional layout layer is formed.

11. Orthosis according to one of the preceding claims, **characterized in that** for increasing a partial stiffness of the support body (19) a plurality of layouts of the fiber bundle (26) is provided spaced closely together, bordering on one another, partially overlapping and/or layered.

12. Orthosis according to one of the preceding claims, **characterized in that** between the first and the at least one other support sections (12, 14) a two-dimensionally tapered connecting section (16) is provided, and a first layout layer (24) is created having fiber bundles (26) oriented in a unidirectional manner, which in the tapered connecting section (16) increasingly reduce their spacing coming from the support section (12, 14) and are arranged lying closely together in the connecting section (16), overlapping partially or lying on top of one another.

13. Orthosis according to one of the preceding claims, **characterized in that** the support body (19) is designed as an ankle-foot orthosis, and the first support section (12) includes at least a foot sole, the at least one other support section (14) having of a lower leg attachment, and the at least one connecting section (16) extends as a brace between the foot sole and the lower leg attachment.

14. Orthosis according to one of the preceding claims, **characterized in that** the connecting section designed as a brace exhibits a distribution of the fiber bundle (26) in such a manner that mechanical properties of the connecting section are obtained that are fitted to the human ankle joint.

15. A method for production of an orthosis having a first support section (12) and at least one other support section (14) forming a spatially shaped support body (19, the support sections being configured to be attached to a patient and to support limbs or upper body sections of a patient, **characterized in that** for production of a reinforcing structure (21) for a support body (19) a continuous laid out fiber bundle (26) formed from reinforcing fibers running parallel to one another is applied to a support layer (23), that the fiber bundle (26) having reinforcing fibers running parallel to one another is laid out, at least in part corresponding to stresses occurring in the support body (19), oriented along lines of stress, wherein the laying out of the fiber bundle (26) is carried out within a surface area (27) of the support layer (23), corresponding to a two-dimensional layout of the support body (19), and that the support layer (23) and the fiber bundle (26) sewn thereto, are spatially shaped and impregnated with a curable base material (38).

16. The method according to claim 15, **characterized in that** the bundle (26) of reinforcing fibers is applied to the support layer, and is sewn or stitched to the support layer (23) during or immediately after application.

17. The method according to claim 15 or 16, **characterized in that** after completion of the application of the fiber bundle (26) to the support layer (23), the support layer is cut to the shape of the surface area (27) corresponding to the two-dimensional layout of the support body (19), and preferably is subsequently positioned on a positive model (35) for lamination with the curable base material (38).

18. The method according to claim 15, 16 or 17, **characterized in that** one or more layout layers (24, 25) are applied to the support layer.

## Revendications

1. Orthèse comportant une première partie de support (12) et au moins une autre partie de support (14), lesquelles forment un corps de support (19) façonné en trois dimensions, les parties de support (12, 14) étant conçues pour s'appuyer contre le patient et pour supporter des membres ou des parties du corps du patient,
**caractérisée en ce que**
- le corps de support (19) formé de la première partie de support et d'au moins une autre partie de support (12, 14) est composé d'une structure de renforcement (21) comprenant une couche porteuse (23) sur laquelle est appliqué un faisceau de fibres (26) posé en continu, lequel est constitué de fibres de renforcement s'étendant parallèlement les unes aux autres,
- une pose du faisceau de fibres (26) comprenant des fibres de renforcement s'étendant parallèlement les unes aux autres est orientée au moins en majeure partie selon les lignes de force en fonction des tensions apparaissant dans le corps de support (19),
- la pose du faisceau de fibres (26) est réalisée à l'intérieur d'une surface (27) sur la couche porteuse (23), laquelle correspond à une surface développée du corps de support (19) façonné en trois dimensions, et **en ce que**
- la structure de renforcement (21) façonnée en trois dimensions et imprégnée d'une matière de base durcissable forme le corps de support (19).

2. Orthèse selon la revendication 1, **caractérisée en ce que** la surface de la couche porteuse, laquelle correspond à une surface développée du corps de support, est recouverte sensiblement au moins en majeure partie et de préférence entièrement par le faisceau de fibres (26).

3. Orthèse selon la revendication 1 ou 2, **caractérisée en ce que** l'application du faisceau de fibres (26) est réalisée par couture ou par broderie au moyen d'un fil de fixation ou par collage au moyen d'un adhésif.

4. Orthèse selon la revendication 1, 2 ou 3, **caractérisée en ce que** le faisceau de fibres (26) est composé de 3 000 à 56 000 et de préférence de 12 000 à 48 000 et plus préférentiellement de 24 000 à 36 000 fibres.

5. Orthèse selon l'une quelconque des revendications précédentes, **caractérisée en ce que** le faisceau de fibres (26) forme au moins une couche structurale (24, 25) sur la couche porteuse (23), les fibres du faisceau de fibres (26) à l'intérieur de la couche structurale (24, 25) étant orientées de manière unidirectionnelle et/ou en croix les unes par rapport aux autres.

6. Orthèse selon l'une quelconque des revendications précédentes, **caractérisée en ce que** le faisceau de fibres (26) réalisé sous la forme d'un faisceau de fibres marginales (28), en particulier sous la forme d'un faisceau de fibres marginales (28) fermé, est posé le long du bord et/ou parallèlement au bord de la surface (27) de la couche porteuse (23), laquelle correspond à la surface développée du corps de support (19).

7. Orthèse selon l'une quelconque des revendications précédentes, **caractérisée en ce que** le faisceau de fibres marginales (28) présente une largeur dans la plage de 0,3 cm à 3,5 cm et de préférence dans la plage de 1 cm à 2 cm.

8. Orthèse selon l'une quelconque des revendications précédentes, **caractérisée en ce qu'**au moins une partie de liaison (16) est présente entre la première et au moins une autre partie de support (12, 14) et les parties de support (12, 14) sont reliées l'une à l'autre par des faisceaux de fibres (26), au moins une couche structurale (24, 25) s'étendant au moins partiellement dans la partie de support (12, 14) et/ou la partie de liaison (16) et/ou la zone de transition (17) entre la partie de support (12, 14) et la partie de liaison (16).

9. Orthèse selon l'une quelconque des revendications précédentes, **caractérisée en ce qu'**une pose du ou des faisceaux de fibres (26), lesquels sont orientés de manière unidirectionnelle les uns par rapport aux autres et forment une couche structurale de base, est présente à l'intérieur d'une couche structurale.

10. Orthèse selon l'une quelconque des revendications précédentes 1 à 6, **caractérisée en ce qu'**une pose du faisceau de fibres (26) est réalisée n croix à l'intérieur d'une couche structurale (25) et en particulier le tracé du faisceau de fibres (26) est orienté en diagonale par rapport au tracé de ligne de force dans le corps de support (19), si bien qu'une couche structurale à torsion est formée.

11. Orthèse selon l'une quelconque des revendications précédentes, **caractérisée en ce que** pour augmenter une résistance partielle du corps de support (19), plusieurs poses du faisceau de fibres (26) sont à proximité immédiate les unes des autres, adjacentes les unes aux autres, se chevauchent et/ou se recouvrent en partie.

12. Orthèse selon l'une quelconque des revendications précédentes, **caractérisée en ce qu'**une partie de liaison (16) rétrécie en surface est présente entre la première et la ou les autres parties de support (12, 14) et une première couche structurale (24) est constituée de faisceaux de fibres (26) orientés de manière unidirectionnelle, lesquels diminuent progressivement leur écart à partir de la partie de support (12, 14) dans la partie de liaison (16) rétrécie et se situent à proximité étroite les uns des autres, se chevauchent ou sont superposés partiellement dans la partie de liaison (16).

13. Orthèse selon l'une quelconque des revendications précédentes, **caractérisée en ce que** le corps de support (19) est réalisé sous la forme d'une orthèse cheville-pied et la première partie de support (12) comprend au moins une plante du pied, la ou les autres parties de support (14) comprennent un système de jambe et la ou les parties de liaison (16) s'étendent sous la forme d'un étrier entre la plante du pied et le système de jambe.

14. Orthèse selon l'une quelconque des revendications précédentes, **caractérisée en ce que** la partie de liaison réalisée sous la forme d'un étrier présente une répartition du faisceau de fibres (26) telle que cela permet de procurer à la partie de liaison des propriétés mécaniques adaptées à l'articulation tibio-tarsienne humaine.

15. Procédé permettant de produire une orthèse comportant une première partie de support (12) et au moins une autre partie de support (14) formant un corps de support (19) façonné en trois dimensions, les parties de support (12, 14) étant conçues pour s'appuyer contre le patient et pour supporter des membres ou des parties du corps du patient,
**caractérisé en ce que**
- pour produire une structure de renforcement (21) pour un corps de support (19), un faisceau de fibres (26) posé en continu, lequel est constitué de fibres de renforcement s'étendant parallèlement les unes aux autres, est appliqué sur une couche porteuse (23),
- le faisceau de fibres (26) comprenant des fibres de renforcement s'étendant parallèlement les unes aux autres est posé au moins en partie en étant orienté selon les lignes de force en fonction des tensions apparaissant dans le corps de support (19),
- la pose du faisceau de fibres (26) étant réalisée à l'intérieur d'une surface (27) sur la couche porteuse (23), laquelle correspond à une surface développée du corps de support (19), et **en ce que**
- la couche porteuse (23) sur laquelle est cousu le faisceau de fibres (26) est formée en trois dimensions et imprégnée d'une matière de base (38) durcissable.

16. Procédé selon la revendication 15, **caractérisé en ce que** le faisceau (26) des fibres de renforcement est posé sur la couche porteuse et cousu ou brodé pendant ou directement après avoir été posé sur la couche porteuse (23).

17. Procédé selon la revendication 15 ou 16, **caractérisé en ce qu'**une fois le faisceau de fibres (26) appliqué sur la couche porteuse (23), la surface (27) est découpée, laquelle correspond à la surface développée du corps de support (19), puis est de préférence positionnée sur un modèle positif (35) afin d'être laminée avec la matière de base (38) durcissable.

18. Procédé selon la revendication 15, 16 ou 17, **caractérisé en ce qu'**une ou plusieurs couches structurales (24, 25) sont appliquées sur la couche porteuse.
